(19) Europäisches Patentamt
European Patent Office
Office européen des brevets

(11) Veröffentlichungsnummer : **0 121 176 B1**

(12) **EUROPÄISCHE PATENTSCHRIFT**

(45) Veröffentlichungstag der Patentschrift : **30.09.87**

(51) Int. Cl.⁴ : **C 07 D209/34**, C 07 D403/06, A 61 K 31/40

(21) Anmeldenummer : **84103045.5**

(22) Anmeldetag : **20.03.84**

# (54) Indolinon-(2)-Derivate, Verfahren zu ihrer Herstellung, diese Verbindungen enthaltende Arzneimittel und Zwischenprodukte.

(30) Priorität : **25.03.83 DE 3310891**

(43) Veröffentlichungstag der Anmeldung : **10.10.84 Patentblatt 84/41**

(45) Bekanntmachung des Hinweises auf die Patenterteilung : **30.09.87 Patentblatt 87/40**

(84) Benannte Vertragsstaaten : **AT BE CH DE FR GB IT LI LU NL SE**

(56) Entgegenhaltungen :
**EP-A- 0 071 950**
**WO-A-82 /025 50**
**DE-A- 1 493 853**
**DE-A- 1 593 901**
**DE-A- 2 230 426**
**DE-A- 2 262 285**
**DE-A- 2 331 721**
**DE-A- 2 453 113**
**CHEMICAL ABSTRACTS, Band 82, Nr. 4, 27. Januar 1975, Seite 493, Nr. 31254h, Columbus, Ohio, USA**
**CHEMICAL ABSTRACTS, Band 85, Nr. 10, 6. September 1976, Seite 532, Nr. 77994q, Columbus, Ohio, USA**
**Die Akte enthält technische Angaben, die nach dem Eingang der Anmeldung eingereicht wurden und die nicht in dieser Patentschrift enthalten sind.**

(73) Patentinhaber : **Boehringer Mannheim GmbH**
**Sandhofer Strasse 116**
**D-6800 Mannheim 31 (DE)**

(72) Erfinder : **Michel, Helmut**
**Ziegelgasse 2a**
**D-6800 Mannheim 31 (DE)**
Erfinder : **Marzenell, Klaus**
**Vorderer-Ring 13**
**D-6802 Ladenburg (DE)**
Erfinder : **Kampe, Wolfgang, Dr. rer. nat.**
**Zedernstrasse 49**
**D-6805 Heddesheim (DE)**
Erfinder : **Bartsch, Wolfgang, Dr.**
**Franconviller-Strasse 5**
**D-6806 Viernheim (DE)**
Erfinder : **Schaumann, Wolfgang, Prof. Dr.**
**Mönchhofstrasse 58**
**D-6900 Heidelberg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 121 176 B1

Jouve, 18, rue St-Denis, 75001 Paris, France

**Beschreibung**

Die Erfindung betrifft neue Indolinon-(2)-Derivate der allgemeinen Formel I

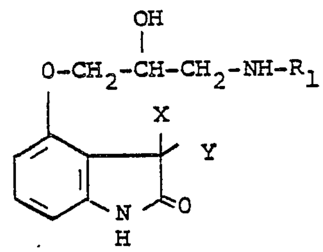

(I)

in welcher

$R_1$ eine $C_1$-$C_6$-Alkylgruppe oder eine Gruppe

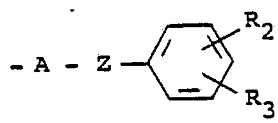

worin

A eine geradkettige oder verzweigte $C_2$-$C_4$-Alkylengruppe und

Z ein Sauerstoff- oder Schwefelatom,

$R_2$ und $R_3$, die gleich oder verschieden sein koennen, jeweils Wasserstoff, Halogen, eine Hydroxygruppe, eine $C_2$-$C_6$-Alkanoylgruppe, eine $C_2$-$C_4$-Alkenylgruppe, eine $C_2$-$C_4$-Alkinylgruppe, eine $C_1$-$C_6$-Alkylgruppe, eine $C_1$-$C_6$-Alkoxygruppe, eine $C_2$-$C_4$-Alkenyloxygruppe, eine $C_2$-$C_4$-Alkinyloxygruppe, eine $C_1$-$C_6$-Alkylthiogruppe, eine $C_2$-$C_6$-Alkanoylaminogruppe oder eine Gruppe

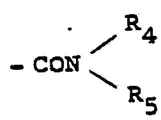

worin $R_4$ und $R_5$ gleich oder verschieden sind und Wasserstoff, einen $C_1$-$C_6$-Alkyl- oder $C_3$-$C_{10}$-Cycloalkylrest darstellen oder $R_4$ und $R_5$ gemeinsam einen gegebenenfalls durch ein Sauerstoff- oder Schwefelatom oder eine Gruppe $>N—R_6$, worin $R_6$ Wasserstoff oder eine $C_1$-$C_6$-Alkylgruppe bedeutet, unterbrochenen $C_2$-$C_8$-Alkylenrest darstellen, bedeuten,

X Wasserstoff und

Y Wasserstoff oder eine Gruppe

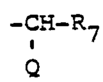

worin

Q Wasserstoff darstellt oder auch gemeinsam mit X eine Bindung bilden kann und $R_7$ einen Furan-, Thiophen-, Pyrrol-, Pyrazol-, Imidazol-, Triazol-, Tetrazol-, Imidazolin-, Pyridin-, Pyrimidin-, Uracil-, Indol- oder Indazol-Rest, der gegebenenfalls ein oder mehrfach durch Hydroxyl oder $C_1$-$C_6$-Alkyl substituiert sein kann oder einen Phenylrest, der gegebenenfalls ein oder mehrfach durch eine Hydroxygruppe, eine Mercaptogruppe, eine Carboxylgruppe, eine $C_1$-$C_6$-Alkoxycarbonylgruppe, eine $C_1$-$C_6$-Alkoxygruppe, eine $C_1$-$C_6$-Alkylthiogruppe, eine $C_1$-$C_6$-Alkylsulfinylgruppe, eine $C_1$-$C_6$-Alkylsulfonylgruppe, eine $C_2$-$C_6$-Alkanoylamidogruppe, eine $C_1$-$C_6$-Alkylsulfonylamidogruppe, eine Nitrogruppe, eine Aminogruppe, Halogen, eine $C_1$-$C_6$-Alkylgruppe, eine Methylendioxygruppe oder eine Cyanogruppe substituiert ist, darstellt, mit der Maßgabe, daß $R_1$ nicht eine $C_1$-$C_6$-Alkylgruppe sein kann, wenn X und Y gleichzeitig Wasserstoff bedeuten,

sowie deren pharmakologisch verträgliche Salze, Verfahren zu ihrer Herstellung und diese Verbindungen enthaltende Arzneimittel.

Da die Verbindungen der allgemeinen Formel I entweder ein asymmetrisches Kohlenstoffatom oder für den Fall, dass Y

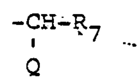

und X und Q Wasserstoff bedeuten, auch zwei asymmetrische Kohlenstoffatome besitzen, sind ferner Gegenstand der Erfindung die optisch aktiven Formen und racemischen Gemische dieser Verbindungen.

Fuer den Fall, daß in Verbindungen der allgemeinen Formel I X gemeinsam mit Q eine Bindung bedeutet, sind ebenfalls deren E-und Z-Isomere Gegenstand der Erfindung.

In der DE-OS 22 30 426 sind Indolinon-(2)-Derivate mit aehnlicher Struktur beschrieben, denen eine Blockerwirkung auf die adrenergischen β-Rezeptoren zugeschrieben wird. Demgegenueber zeigen die Verbindungen der Erfindung ueberraschend zusaetzliche Wirkqualitaeten, und zwar eine akute Senkung des arteriellen Blutdrucks sowie eine bevorzugte Blockade der cardialen β-Rezeptoren. Sie eignen sich daher besonders zur Behandlung oder Prophylaxe bei Herz- und Kreislauferkrankungen sowie bei Bluthochdruck.

Die $C_1$-$C_6$-Alkylgruppen der Substituenten $R_1$, $R_2$, $R_3$, $R_4$, $R_5$, $R_6$ und $R_7$ sind geradkettige oder verzweigte Gruppen wie z. B. der Methyl-, Ethyl-, Propyl-, Isopropyl-, n-Butyl-, iso-Butyl-, tert.-Butyl- oder n-Hexylrest.

Insbesondere kommen jedoch die Methyl-, Ethyl-, Isopropyl- und die tert.-Butylgruppe in Frage.

Cycloalkylreste der Substituenten $R_4$ und $R_5$ sind insbesondere der Cyclopropyl-, Cyclopentyl- oder Cyclohexylrest.

Unter einer $C_2$-$C_6$-Alkanoylgruppe der Substituenten $R_2$ und $R_3$ sind geradkettige Gruppen mit 2-6 Kohlenstoffatomen zu verstehen. Bevorzugt ist der Acetylrest.

Von den $C_2$-$C_4$-Alkenyl- und Alkinylgruppen der Substituenten $R_2$ und $R_3$ sind die Allyl- und Propargylgruppe bevorzugt.

Die $C_1$-$C_6$-Alkoxy- bzw. Alkoxycarbonylgruppen der Substituenten $R_2$, $R_3$ und $R_7$ enthalten vorzugsweise 1-4 Kohlenstoffatome, wie z. B. die Methoxy-, Ethoxy-, Propoxy- und Butoxygruppe.

Von den $C_2$-$C_4$-Alkenyloxy- und Alkinyloxygruppen der Substituenten $R_2$ und $R_3$ sind die Allyloxy- und Propargyloxygruppe bevorzugt.

Von einer $C_1$-$C_6$-Alkylthiogruppe der Substituenten $R_2$, $R_3$ und $R_7$ ist der Methylmercaptorest bevorzugt.

Von einer $C_1$-$C_6$-Alkylsulfenyl-, Alkylsulfinyl- bzw. Alkylsulfonylamidogruppe des Substituenten $R_7$ ist der Methylsulfenyl-, Methylsulfinyl- bzw. Methylsulfonamido-Rest bevorzugt.

Unter einer $C_2$-$C_6$-Alkanoylamidogruppe ist vorzugsweise der Acetamidorest zu verstehen.

Unter einer $C_2$-$C_8$-Alkylengruppe der Reste $R_4$ und $R_5$ sind geradkettige oder verzweigte Gruppen mit vorzugsweise 2-5 Kohlenstoffatomen, wie z. B. die Ethylen-, Propylen-, n-Butylen- und n-Pentylengruppe, zu verstehen. Mit dem Stickstoffatom können Heterocyclen wie z. B. Aziridin, Azetidin, Pyrrolidin, Piperidin, Morpholin, Piperazin gebildet werden.

Unter Halogen wird im Sinne der Erfindung Fluor, Chlor, Brom und Jod verstanden, vorzugsweise Fluor, Chlor und Brom.

Die Herstellung der Verbindungen der allgemeinen Formel I ist dadurch gekennzeichnet, daß man in an sich bekannter Weise entweder

a) eine Verbindung der allgemeinen Formel II

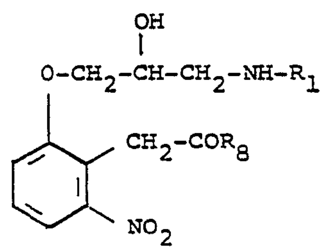

(II)

in welcher $R_1$ die angegebene Bedeutung hat und $R_8$ eine abspaltbare Gruppe darstellt, reduziert und cyclisiert und gewuenschtenfalls mit einer Verbindung der allgemeinen Formel III

$$O = CH - R_7 \qquad (III)$$

in welcher $R_7$ die angegebene Bedeutung hat oder einem reaktiven Derivat hiervon, umsetzt oder

b) eine Verbindung der allgemeinen Formel IV

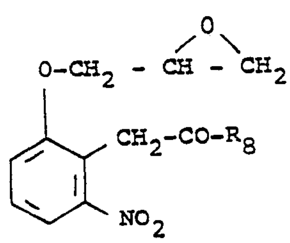

(IV)

in welcher $R_8$ die angegebene Bedeutung hat, reduziert und cyclisiert, gewuenschtenfalls mit einer Verbindung der allgemeinen Formel III umsetzt, die dabei erhaltene Verbindung der allgemeinen Formel V

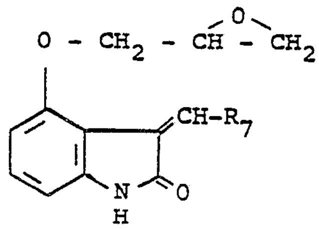

(V)

in welcher $R_7$ die angegebene Bedeutung hat mit einer Verbindung der allgemeinen Formel VI

$$H_2N - R_1 \qquad (VI)$$

in welcher $R_1$ die angegebene Bedeutung hat umsetzt, gegebenenfalls anschließend eine Verbindung der allgemeinen Formel I, in der X und Q eine Bindung bilden, nach bekannten Methoden in eine Verbindung der allgemeinen Formel I, in der X und Q Wasserstoff bedeuten, umwandelt und gewuenschtenfalls in ein pharmakologisch vertraegliches Salz ueberfuehrt.

Die Reduktionen nach den Verfahren a) und b) können mit katalytisch angeregtem Wasserstoff durchgeführt werden. Vorzugsweise wird Palladium/Kohle oder Raney-Nickel in Methanol verwendet, wobei bei Temperaturen von 0-100 °C gearbeitet werden kann. Die Cyclisierung geschieht in saurem Milieu, vorteilhaft in essigsaurer Lösung.

Die Überführung von Verbindungen der allgemeinen Formel I, in der X und Q zusammen eine Bindung bilden, in Verbindungen der allgemeinen Formel I, in der X und Q Wasserstoff bedeuten, geschieht vorzugsweise durch katalytische Hydrierung mit Wasserstoff in Gegenwart von Edelmetallkatalysatoren wie z. B. Palladium/Kohle, Platinoxid etc. in einem Lösungsmittel wie Methanol oder Ethanol unter Zusatz von Triethylamin.

Abspaltbare Gruppen $R_8$ in Verbindungen der allgemeinen Formeln II und IV sind Amino-, Imidazolyl-, Hydroxy-, oder $C_1$-$C_6$-Alkoxuppen, vorzugsweise Hydroxy-, Methoxy-, Ethoxy- und Propoxygruppen.

Verbindungen der allgemeinen Formel II können durch Umsetzung von Verbindungen der Formel IV mit Verbindungen der Formel VI erhalten werden. Sie sind, wenn $R_1$ nicht $C_1$-$C_6$-Alkyl bedeutet, neu.

Die Herstellung der Verbindungen der allgemeinen Formel IV ist in der EP-A-00 14 928 beschrieben.

Verbindungen der Formel V sind neu. Gegenstand der Erfindung sind demnach auch neue Zwischenprodukte der allgemeinen Formel V zur Herstellung von Verbindungen der allgemeinen Formel I.

Die Umsetzungen der Vorstufen mit Verbindungen der allgemeinen Formel III koennen ohne Loesungsmittel oder in einem inerten Loesungsmittel, wie z. B. Methanol, Ethanol, n-Butanol, Diethylether, Methylenchlorid, Toluol, Essigester, Tetrahydrofuran, Dioxan, Dimethylformamid oder Dimethylsulfoxid unter Zusatz eines geeigneten Katalysators, wie z. B. Ammoniak, Triethylamin, N-Ethyl-diisopropylamin, Tributylamin, Piperidin, Morpholin, 1-Methylpiperidin, 4-Methylmorpholin oder Natriummethylat durchgefuehrt werden. Besonders geeignet sind jedoch Methanol, Ethanol und Dimethylsulfoxid sowie Triethylamin, Piperidin und 1-Methylpiperidin.

Die erfindungsgemaessen Verbindungen der allgemeinen Formel I koennen in Form eines racemischen Gemisches anfallen. Die Trennung des Racemats in die optisch aktiven Formen geschieht nach an sich bekannten Methoden ueber die diastereomeren Salze aktiver Säuren, wie z. B. Weinsaeure, Aepfelsaeure oder Camphersulfonsaeure.

Zur Uberfuehrung der Verbindungen der allgemeinen Formel I in ihre pharmakolgisch unbedenklichen Salze setzt man diese, vorzugsweise in einem organischen Loesungsmittel, mit der aequivalenten Menge einer anorganischen oder organischen Saeure, z. B. Salzsaeure, Bromwasserstoffsaeure, Phosphorsaeure, Schwefelsäure, Essigsaeure, Citronensaeure, Weinsaeure, Maleinsaeure, Fumarsaeure, Benzoesaeure und Cyclohexylsulfaminsaeure um.

Zur Herstellung von Arzneimitteln werden die Substanzen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z. B. Olivenoel, suspendiert oder geloest.

Die erfindungsgemaessen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionslösungen, ueblichen Zusaetze wie Stabilisierungsmittel, Loesungsvermittler oder Puffer enthaelt.

Derartige Zusaetze sind z. B. Tartrat- und Citratpuffer, Ethanol, Komplexbildner (wie Ethylendiamintetraessigsaeure und deren nichttoxische Salze), hochmolekulare Polymere (wie fluessiges Polyethylenoxid) zur Viskositaetsregulierung. Feste Traegerstoffe sind z. B. Staerke, Lactose, Mannit, Methylcellulose,

Talkum, hochdisperse Kieselsaeuren, hochmolekulare Fettsaeuren (wie Sterinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks- und Sueßstoffe enthalten.

Bevorzugt im Sinne der Erfindung sind außer den in den Beispielen genannten Verbindungen die folgenden :

4-[2-Hydroxy-3-[2-(3-carbamoyl-4-hydroxyphenoxy) ethylamino]-propoxy]-3-(2-hydroxybenzyliden) indolinon (2)

4-[2-Hydroxy-3-[2-(3-carbamoyl-4-hydroxyphenoxy) ethylamino]-propoxy]-3-(pyrazol-5-yl) methylen-indolinon (2)

4-[2-Hydroxy-3-(2-phenoxyethylamino) propoxy]-3-(imidazol-5-yl)-methylen-indolinon (2)

4-[2-Hydroxy-3-(2-phenoxyethylamino) propoxy]-3-(4-methyl-imidazolin-2-on-5yl) methylen-indolinon (2)

4-[2-Hydroxy-3-[2-(4-N-methyl-carbamoylphenoxy) ethylamino]-propoxy]-3-(pyrazol-5-yl) methylen-indolinon (2)

4-[2-Hydroxy-3-[2-(4-N-n-butyl-carbamoylphenoxy) ethylamino]-propoxy]-3-(pyrazol-5-yl) methylen-indolinon (2)

4-[2-Hydroxy-3-[2-(4-morpholinocarbonylphenoxy) ethylamino]-propoxy]-3-(pyrazol-5-yl) methylen-indolinon (2)

4-[2-Hydroxy-3-[2-(4-N-methylpiperazinocarbonylphenoxy) ethylamino]-propoxy]-3-(pyrazol-5-yl) methylen-indolinon (2)

4-[2-Hydroxy-3-(2-phenoxyethylamino) propoxy-3-(2-mercaptobenzyliden) indolinon (2)

4-[2-Hydroxy-3-(4-phenoxybutylamino) propoxy]-3-(pyrazol-5-yl) methylen-indolinon (2)

4-[2-Hydroxy-3-(1,1-dimethyl-2-phenoxy-ethylamino) propoxy]-3- pyrazol-5-yl) methylen-indolinon (2)

4-[2-Hydroxy-3-[2-(2-methoxy-phenylmercapto) ethylamino]-propoxy]-3-(pyrazol-5-yl) methylen-indolinon (2)

4-[2-Hydroxy-3-[2-(4-hydroxy-phenoxy) ethylamino] propoxy]-3-(3-chlor-benzyliden) indolinon (2)

4-[2-Hydroxy-3-[2-(4-hydroxy-phenoxy) ethylamino] propoxy]-3-(2-methyl-benzyliden) indolinon (2)

4-[2-Hydroxy-3-[2-(4-hydroxy-phenoxy) ethylamino] propoxy]-3-(3,4-methylendioxy-benzyliden) indolinon (2)

4-[2-Hydroxy-3-[2-(4-hydroxy-phenoxy) ethylamino] propoxy]-3-(4-cyano-benzyliden) indolinon. (2)

Erfindungsgemäße Zwischenprodukte, die durch Umsetzung von 4-(2,3-Epoxy-propoxy) indolinon (2) mit entsprechenden Aldehyden erhalten werden, sind unter anderem :

4-(2,3-Epoxy-propoxy)-3-(imidazol-5-yl) methylen-indolinon (2)
4-(2,3-Epoxy-propoxy)-3-(1,2,4-triazol-3-yl) methylen-indolinon (2)
4-(2,3-Epoxy-propoxy)-3-(4-methyl-imidazolin-2-on-5-yl) methylen-indolinon (2)
4-(2,3-Epoxy-propoxy)-3-benzyliden-indolinon (2)
4-(2,3-Epoxy-propoxy)-3-(4-methoxybenzyliden) indolinon (2)
4-(2,3-Epoxy-propoxy)-3-(3,4-dimethoxybenzyliden) indolinon (2)
4-(2,3-Epoxy-propoxy)-3-(2-hydroxy-4-methylmercapto-benzyliden)-indolinon (2)
4-(2,3-Epoxy-propoxy)-3-(pyrrol-2-yl) methylen-indolinon (2)
4-(2,3-Epoxy-propoxy)-3-(imidazol-2-yl) methylen-indolinon (2)
4-(2,3-Epoxy-propoxy)-3-(pyridyl-2-yl) methylen-indolinon (2)
4-(2,3-Epoxy-propoxy)-3-(thiophen-2-yl) methylen-indolinon (2)
4-(2,3-Epoxy-propoxy)-3-(2-acetamido-benzyliden) indolinon (2)
4-(2,3-Epoxy-propoxy)-3-(2-methansulfonylamido-benzyliden)-indolinon (2)
4-(2,3-Epoxy-propoxy)-3-(2-carboxybenzyliden) indolinon (2)
4-(2,3-Epoxy-propoxy)-3-(indol-2-yl) methylen-indolinon (2)
4-(2,3-Epoxy-propoxy)-3-(2-nitrobenzyliden) indolinon (2)
4-(2,3-Epoxy-propoxy)-3-(uracil-4-yl) methylen-indolinon (2)
4-(2,3-Epoxy-propoxy)-3-(indazol-3-yl) methylen-indolinon (2)
4-(2,3-Epoxy-propoxy)-3-(2-hydroxy-4-methylsulfinyl-benzyliden)-indolinon (2)
4-(2,3-Epoxy-propoxy)-3-(3-Chlorbenzyliden) indolinon (2)
4-(2,3-Epoxy-propoxy)-3-(2-Methylbenzyliden) indolinon (2)
4-(2,3-Epoxy-propoxy)-3-(3,4-Methylendioxybenzyliden) indolinon (2)
4-(2,3-Epoxy-propoxy)-3-(4-Cyanobenzyliden) indolinon (2)

## Beispiel 1

4-(2-Hydroxy-3-isopropylamino-propoxy)-3-benzyliden-indolinon (2)

2.5 g 4-[2-Hydroxy-3-(isopropylamino) propoxy] indolinon (2) werden in 50 ml Ethanol mit 5 Tropfen

Piperidin und 1.3 ml Benzaldehyd 5 h zum Rueckfluß erhitzt. Nach Einengen der Loesung wird in verd. Milchsaeure und Ether geloest, die waessrige Phase mit Kaliumcarbonat alkalisch gestellt und nach Absaugen der Base aus Isopropanol umkristallisiert. Es werden 2.0 g 4-(2-Hydroxy-3-isopropylamino-propoxy)-3-benzyliden-indolinon (2) vom Schmp. 163-165 °C, d. s. 56 % d. Th. erhalten.

Analog zu Beispiel 1 erhaelt man :

(Siehe Tabelle Seite 7 ff.)

| Bezeichnung<br>Schmelzpunkt C° / Lösungsmittel | Ausbeute<br>% |
|---|---|
| a) 4-(2-Hydroxy-3-isopropylamino-propoxy)-3-(2-hydroxybenzyliden)indolinon(2)<br>181-183 / Isopropanol<br>aus<br>4-(2-Hydroxy-3-isopropylamino-propoxy)-indolinon(2)<br>und<br>Salicylaldehyd | 47 |
| b) 4-(2-Hydroxy-3-isopropylamino-propoxy)-3-(4-methoxybenzyliden)indolinon (2)<br>153-156 / Essigester<br>aus<br>4-(2-Hydroxy-3-isopropylamino-propoxy)indolinon(2)<br>und<br>4-Methoxybenzaldehyd | 23 |
| c) 4-(2-Hydroxy-3-isopropylamino-propoxy)-3-(3.4-dimethoxybenzyliden)indolinon(2)<br>173-174 / Ethanol<br>aus<br>4-(2-Hydroxy-3-isopropylamino-propoxy)-indolinon(2)<br>und<br>3.4-Dimethoxybenzaldehyd | 72 |
| d) 4-(2-Hydroxy-3-isopropylamino-propoxy)-3-(2-hydroxy-4-methylmercapto-benzyliden)-indolinon(2)<br>132-133 / Essigester<br>aus<br>4-(2-Hydroxy-3-isopropylamino-propoxy)-indolinon(2)<br>und<br>2-Hydroxy-4-methylmercapto-benzaldehyd | 41 |
| e) 4-(2-Hydroxy-3-isopropylamino-propoxy)-3-(pyrrol-2-yl)methylen-indolinon(2)<br>191-193 / Ethanol<br>aus<br>4-(2-Hydroxy-3-isopropylamino-propoxy)-indolinon(2)<br>und<br>Pyrrol-2-aldehyd | 60 |
| f) 4-(2-Hydroxy-3-isopropylamino-propoxy)-3-(pyrazol-5-yl)methylen-indolinon(2)<br>210-211 / Ethanol<br>aus<br>4-(2-Hydroxy-3-isopropylamino-propoxy)-indolinon(2)<br>und<br>Pyrazol-5-aldehyd | 64 |

| Bezeichnung<br>Schmelzpunkt C° / Lösungsmittel | Ausbeute<br>% |
|---|---|
| g) 4-(2-Hydroxy-3-isopropylamino-propoxy)-3-(imidazol-2-yl)methylen-indolinon(2)<br>223-225 / Methanol<br>aus<br>4-(2-Hydroxy-3-isopropylamino-propoxy)-indolinon(2)<br>und<br>Imidazol-2-aldehyd | 20 |
| h) 4-(2-Hydroxy-3-isopropylamino-propoxy)-3-(pyridyl-2-)methylen-indolinon(2)-benzoat<br>180-181 / Isopropanol<br>aus<br>4-(2-Hydroxy-3-isopropylamino-propoxy)-indolinon(2)<br>und<br>Pyridin-2-aldehyd | 20 |
| i) 4-(2-Hydroxy-3-isopropylamino-propoxy)-3-(thiophen-2-yl)methylen-indolinon(2)<br>180-181 / Ethanol<br>aus<br>4-(2-Hydroxy-3-isopropylamino-propoxy)-indolinon(2)<br>und<br>Thiophen-2-aldehyd | 48 |
| j) 4-(2-Hydroxy-3-isopropylamino-propoxy)-3-(2-acetamido-benzyliden)indolinon(2)<br>174-176 / Isopropanol<br>aus<br>4-(2-Hydroxy-3-isopropylamino-propoxy)-indolinon(2)<br>und<br>2-Acetamidobenzaldehyd | 35 |

| Bezeichnung<br>Schmelzpunkt C° / Lösungsmittel | Ausbeute<br>% |
|---|---|
| k) 4-(2-Hydroxy-3-isopropylamino-propoxy)-3-(2-methansulfonylamido-benzyliden)-indolinon(2)<br>207-208 / Essigester<br>aus<br>4-(2-Hydroxy-3-isopropylamino-propoxy)-indolinon(2)<br>und<br>2-Methansulfonylamidobenzaldehyd | 47 |
| l) 4-[2-Hydroxy-3-(2-phenoxyethylamino)-propoxy] 3-(2-hydroxybenzyliden)indolinon(2)<br>130-132 / Essigester<br>aus<br>4-[2-Hydroxy-3-(2-phenoxyethylamino)propoxy]-indolinon(2) (Beisp. 2)<br>und<br>Salicylaldehyd | 38 |
| m) 4-<2-Hydroxy-3-[2-(2-methoxyphenoxy)ethyl-amino]propoxy>-3-(2-hydroxybenzyliden)-indolinon(2)<br>171-172 / Ethanol<br>aus<br>4-<2-Hydroxy-3-[2-(2-methoxyphenoxy)ethyl-amino]propoxy>indolinon(2) (Beisp. 2 a)<br>und<br>Salicylaldehyd | 48 |
| n) 4-<2-Hydroxy-3-[2-(2-allyloxyphenoxy)ethyl-amino]propoxy>-3-(2-hydroxybenzyliden)-indolinon(2)<br>147-148 / Ethanol<br>aus<br>4-<2-Hydroxy-3-[2-(2-allyloxyphenoxy)ethyl-amino]propoxy>indolinon(2) (Beisp. 3)<br>und<br>Salicylaldehyd | 42 |

| Bezeichnung<br>Schmelzpunkt C° / Lösungsmittel | Ausbeute<br>% |
|---|---|
| o) 4-<2-Hydroxy-3-[2-(2-methylmercaptophenoxy)-ethylamino]propoxy>-3-(2-hydroxybenzyliden)-indolinon(2)benzoat<br>177 / Ethanol<br>aus<br>4-<2-Hydroxy-3-[2-methylmercaptophenoxy)-ethylamino]propoxy>indolinon(2) (Beisp. 3a)<br>und<br>Salicylaldehyd | 20 |
| p) 4-<2-Hydroxy-3-[2-(4-hydroxyphenoxy)ethyl-amino]propoxy>-3-(2-hydroxybenzyliden)-indolinon(2)benzoat<br>187-188 / Isopropanol<br>aus<br>4-<2-Hydroxy-3-[2-(4-hydroxyphenoxy)ethyl-amino]propoxy>indolinon(2) (Beisp. 2b)<br>und<br>Salicyclaldehyd | 42 |
| q) 4-[2-Hydroxy-3-(2-phenoxyethylamino)propoxy]-3-(2-carboxybenzyliden)indolinon(2)<br>238-240 / Wasser<br>aus<br>4-[2-Hydroxy-3-(2-phenoxyethylamino)-propoxy]indolinon(2) (Beisp. 2)<br>und<br>2-Carboxybenzaldehyd | 84 |
| r) 4-<2-Hydroxy-3-[2-(4-hydroxyphenoxy)ethyl-amino]propoxy>-3-(2-carboxybenz-yliden)indolinon(2)<br>260 / Ethanol<br>aus<br>4-<2-Hydroxy-3-[2-(4-hydroxyphenoxy)ethyl-amino]propoxy>indolinon(2) (Beisp. 2b)<br>und<br>2-Carboxybenzaldehyd | 70 |

| Bezeichnung<br>Schmelzpunkt C° / Lösungsmittel | Ausbeute % |
|---|---|
| s) 4-<2-Hydroxy-3-[2-(4-hydroxyphenoxy)ethyl-amino]propoxy>-3-(2-methansulfonylamido-benzyliden)indolinon(2)<br>153-158 / Essigester<br>aus<br>4-<2-Hydroxy-3-[2-(4-hydroxyphenoxy)ethyl-amino]propoxy>indolinon(2) (Beisp. 2b)<br>und<br>2-Methansulfonylamidobenzaldehyd | 44 |
| t) 4-<2-Hydroxy-3-[2-(4-hydroxyphenoxy)ethyl-amino]propoxy>-3-(pyrrol-2-yl)methylen-indolinon(2)<br>205 / Ethanol<br>aus<br>4-<2-Hydroxy-3-[2-(4-hydroxyphenoxy)ethyl-amino]propoxy>indolinon(2) (Beisp. 2b)<br>und<br>Pyrrol-2-aldehyd | 64 |
| u) 4-[2-Hydroxy-3-(2-phenoxyethylamino)propoxy]-3-(pyrazol-5-yl)methylen-indolinon(2)<br>161-162 / Ethanol<br>aus<br>4-[2-Hydroxy-3-(2-phenoxyethylamino)-propoxy]indolinon(2) (Beisp. 2)<br>und Pyrazol-5- aldehyd | 57 |
| v) 4-<2-Hydroxy-3-[2-(4-hydroxyphenoxy)ethyl-amino]propoxy>-3-(pyrazol-5-yl)methylen-indolinon(2)<br>178-180 / Ethanol<br>aus<br>4-<2-Hydroxy-3-[2-(4-hydroxyphenoxy)ethyl-amino]propoxy>indolinon(2) (Beisp. 2b)<br>und<br>Pyrazol-5-aldehyd | 64 |

| Bezeichnung<br>Schmelzpunkt C° / Lösungsmittel | Ausbeute % |
|---|---|
| w) 4-[2-Hydroxy-3-(2-phenoxyethylamino)propoxy]-3-(indol-2-yl)methylen-indolinon(2)<br>204-205 / n-Butanol<br>aus<br>4-[2-Hydroxy-3-(2-phenoxyethylamino)propoxy]-indolinon(2) (Beisp. 2)<br>und<br>Indol-2-aldehyd | 74 |
| x) 4-[2-Hydroxy-3-(2-phenoxyethylamino)propoxy]-3-(2-nitrobenzyliden)indolinon(2)<br>159-161 / Essigester<br>aus<br>4-[2-Hydroxy-3-(2-phenoxyethylamino)propoxy]-indolinon(2) (Beisp. 2)<br>und<br>2-Nitrobenzaldehyd | 40 |
| y) 4-[2-Hydroxy-3-(2-phenoxyethylamino)-propoxy]-3-(uracil-4-yl)methylen-indolinon(2)<br>216-218 / Essigester<br>aus<br>4-[2-Hydroxy-3-(2-phenoxyethylamino)propoxy]-indolinon(2) (Beisp. 2)<br>und<br>Uracil-4-aldehyd | 46 |
| z) 4-[2-Hydroxy-3-(2-phenoxyethylamino)-propoxy]-3-(indazol-3-yl)methylen-indolinon(2)<br>192-194 / Essigester<br>aus<br>4-[2-Hydroxy-3-(2-phenoxyethylamino)-propoxy]-indolinon(2) (Beisp. 2)<br>und<br>Indazol-3-aldehyd | 27 |

| Bezeichnung<br>Schmelzpunkt C° / Lösungsmittel | Ausbeute<br>% |
|---|---|
| z 1) 4-[2-Hydroxy-3-(2-phenoxyethylamino)propoxy]-3-(1.2.4-triazol-3-yl)methylen-indolinon(2)<br>177-180 / Ethanol<br>aus<br>4-[2-Hydroxy-3-(2-phenoxyethylamino)propoxy]-indolinon(2) (Beisp. 2)<br>und<br>1.2.4-Triazol-3-aldehyd | 28 |
| z 2) 4-<2-Hydroxy-3-[2-(4-carbamoylphenoxy)ethylamino]propoxy>-3-(pyrazol-5-yl)methylen-indolinon(2)<br>158-160 / Methanol<br>aus<br>4-<2-Hydroxy-3-[2-(4-carbamoylphenoxy)ethylamino]propoxy>indolinon(2) (Beisp. 2c)<br>und<br>Pyrazol-5-aldehyd | 54 |
| z 3) 4-<2-Hydroxy-3-[2-(4-N-isopropyl-carbamoylphenoxy)ethylamino]propoxy>-3-(pyrazol-5-yl)-methylen-indolinon(2)<br>217-219 / Methanol<br>aus<br>4-<2-Hydroxy-3-[2-(4-N-isopropyl-carbamoylphenoxy)ethylamino]propoxy>indolinon(2) (Beisp. 2d)<br>und<br>Pyrazol-5-aldehyd | 70 |
| z 4) 4-<2-Hydroxy-3-[2-(4-N-cyclopentyl-carbamoylphenoxy)ethylamino]propoxy>-3-(pyrazol-5-yl)-methylen-indolinon(2)<br>195-198 / Ethanol<br>aus<br>4-<2-Hydroxy-3-[2-(4-N-cyclopentyl-carbamoylphenoxy)ethylamino]propoxy>indolinon(2) (Beisp. 2e)<br>und<br>Pyrazol-5-aldehyd | 64 |

| Bezeichnung<br>Schmelzpunkt C° / Lösungsmittel | Ausbeute<br>% |
|---|---|
| z 5) 4-<2-Hydroxy-3-[2-(4-N,N-dimethyl-carbamoyl-phenoxy)ethylamino]propoxy>-3-(pyrazol-5-yl)-methylen-indolinon(2)<br>128-131 / Ethanol<br>aus<br>4-<2-Hydroxy-3-[2-(4-N,N-dimethyl-carbamoyl-phenoxy)ethylamino]propoxy>indolinon(2) (Beisp. 2f)<br>und<br>Pyrazol-5-aldehyd | 31 |
| z 6) 4-[2-Hydroxy-3-(2-phenoxyethylamino)propoxy]-3-(2-aminobenzyliden)indolinon(2)<br>108-112 / Ethanol<br>aus<br>4-[2-Hydroxy-3-(2-phenoxyethylamino)propoxy]-indolinon(2) (Beisp. 2)<br>und<br>2-Aminobenzaldehyd | 10 |
| z 7) 4-<2-Hydroxy-3-[2-(4-hydroxyphenoxy)ethyl-amino]propoxy>-3-(1,2,4-triazol-3-yl)methylen-indolinon(2)<br>233-235 / Ethanol<br>aus<br>4-<2-Hydroxy-3-[2-(4-hydroxyphenoxy)ethyl-amino]-propoxy>indolinon(2) (Beisp. 2b)<br>und<br>1,2,4-Triazol-3-aldehyd | 28 |

Die zur Herstellung der vorstehenden Verbindungen benoetigten Ausgangsstoffe koennen wie folgt hergestellt werden :

4-(2-Hydroxy-3-isopropylamino-propoxy) indolinon (2)

14.0 g 2-(2.3-Epoxy-propoxy)-6-nitrophenylessigsaeureethylester (EP-A-00 14 928) werden in 140 ml Isopropylamin geloest und 3 d bei Raumtemperatur stehen gelassen. Nach Entfernen des ueberschuessigen Amins wird in Ether und verd. Milchsaeure geloest, die waessrige Phase mit Kaliumcarbonat alkalisch gestellt und mit Ether extrahiert. Nach Trocknen und Eindampfen verbleiben 16 g als braeunliches Oel, welches sofort in 150 ml Methanol und 150 ml Essigsaeure bei Raumtemperatur und 1 bar Wasserstoffdruck ueber 10 proz. Palladiumkohle hydriert wird. Nach Absaugen des Katalysators wird im Vakuum abdestilliert und der verbleibende Rueckstand in Wasser geloest und filtriert. Durch Zugabe von Kaliumcarbonat wird die Base ausgefaellt und abgesaugt. Nach Trocknen verbleiben 10.4 g 4-(2-Hydroxy-3-isopropylamino-propoxy)-indolinon (2) vom Schmp. 173-175 °C, d. s. 89 % d. Th.

In analoger Weise koennen durch Umsetzung mit Aminen und anschließender Hydrierung und Cyclisierung die folgenden Verbindungen erhalten werden :

(Siehe Beispiel 2 Seite 15 ff.)

Beispiel 2

| Bezeichnung<br>Schmelzpunkt C° / Lösungsmittel | Ausbeute<br>% |
|---|---|
| 4-[2-Hydroxy-3-(2-phenoxyethylamino)propoxy]-indolinon(2)<br>143-145 / Essigester<br>aus<br>2-(2.3-Epoxy-propoxy)-6-nitrophenylessigsaeureethylester<br>und<br>2-Phenoxyethylamin | 75 |
| a) 4-<2-Hydroxy-3-[2-(2-methoxyphenoxy)ethylamino]propoxy>indolinon(2)<br>156-158 / Essigester<br>aus<br>2-(2.3-Epoxy-propoxy)-6-nitrophenylessigsaeureethylester<br>und<br>2-(2-Methoxyphenoxy)ethylamin | 89 |
| b) 4-<2-Hydroxy-3-[2-(4-hydroxyphenoxy)ethylamino]-propoxy>indolinon(2)<br>186-189 / Ethanol<br>aus<br>2-(2.3-Epoxy-propoxy-6-nitrophenylessigsaeureethylester<br>und<br>N-Benzyl-2-(4-benzyloxyphenoxy)-ethylamin | 80 |

(Siehe Fortsetzung Seite 16 f.)

(Fortsetzung)

| Bezeichnung<br>Schmelzpunkt C° / Lösungsmittel | Ausbeute<br>% |
|---|---|
| c) 4-<2-Hydroxy-3-[2-(4-carbamoylphenoxy)ethylamino]propoxy>indolinon(2)<br>143-147 / Essigester<br>aus<br>2-(2.3-Epoxy-propoxy)-6-nitrophenylessigsaeureethylester<br>und<br>2-(4-Carbamoylphenoxy)ethylamin | 66 |
| d) 4-<2-Hydroxy-3-[2-(4-N-isopropyl-carbamoylphenoxy)ethylamino]propoxy>indolinon(2)<br>175-178 / Essigester<br>aus<br>2-(2.3-Epoxy-propoxy)-6-nitrophenylessigsaeure-ethylester<br>und<br>2-(4-N-Isopropyl-carbamoylphenoxy)ethylamin | 48 |
| e) 4-<2-Hydroxy-3-[2-(4-N-cyclopentyl-carbamoylphenoxy)ethylamino]propoxy>indolinon(2)<br>180-184 / Essigester<br>aus<br>2-(2.3-Epoxy-propoxy)-6-nitrophenylessigsaeure-ethylester<br>und<br>2-(4-N-Cyclopentyl-carbamoylphenoxy)ethylamin | 61 |
| f) 4-<2-Hydroxy-3-[2-(4-N,N-dimethyl-carbamoylphenoxy)ethylamino]propoxy>indolinon(2)<br>131-134 / Essigester<br>aus<br>2-(2.3-Epoxy-propoxy)-6-nitrophenylessigsaeure-ethylester<br>und<br>2-(4-N,N-Dimethyl-carbamoylphenoxy)ethylamin | 48 |

Beispiel 3

4-<2-Hydroxy-3-[2-(2-allyloxyphenoxy) ethylamino] propoxy>-indolinon (2)

4.1 g 4-(2.3-Epoxy-propoxy) indolinon (2) (Beispiel 6) werden in 200 ml n-Butanol geloest, 11.6 g 2-(2-Allyloxyphenoxy) ethyl-amin zugegeben und 2 d bei Raumtemperatur geruehrt. Nach Abdestillieren des n-Butanols wird aus Essigester umkristallisiert. Man erhaelt 6.2 g der Titelverbindung vom Schmp. 148-149 °C, d. s. 77 % d. Th.

Analog Beispiel 3 erhaelt man :

(Siehe Tabelle Seite 17 f.)

| Bezeichnung<br>Schmelzpunkt C° / Lösungsmittel | Ausbeute % |
|---|---|
| a) 4-<2-Hydroxy-3-[2-(2-methylmercaptophenoxy)-ethylamino]propoxy>indolinon(2)<br>173-175 / Essigester<br>aus<br>4-(2.3-Epoxy-propoxy)indolinon(2) (Beisp. 6)<br>und<br>2-(2-Methylmercaptophenoxy)ethylamin | 85 |

Beispiel 4

4-(2-Hydroxy-3-isopropylamino-propoxy)-3-(pyrazol-5-yl)-methylen-indolinon (2)

2.8 g 4-(2.3-Epoxy-propoxy)-3-(pyrazol-5-yl)-methylen-indolinon (2) (Beispiel 5) werden in 25 ml n-Butanol und 25 ml Isopropylamin 2 d bei Raumtemperatur geruehrt. Nach Entfernen des Loesungsmittels wird mit Essigester und Wasser aufgenommen und abgesaugt. Nach Trocknen verbleiben 1.9 g der Titelverbindung vom Schmp. 209-211 °C, d. s. 45 % d. Th.

Analog Beispiel 4 erhaelt man :

| Bezeichnung<br>Schmelzpunkt C° / Lösungsmittel | Ausbeute % |
|---|---|
| a) 4-[2-Hydroxy-3-(2-phenoxy-ethylamino)propoxy]-3-(pyrazol-5-yl)methylen-indolinon(2)<br>161-162 / Essigester/Ethanol<br>aus<br>4-(2.3-Epoxy-propoxy)-3-(pyrazol-5-yl)-methylen-indolinon(2) (Beisp. 5)<br>und<br>2-Phenoxy-ethylamin | 40 |

Die in Beispiel 4 benoetigten Ausgangsstoffe koennen wie folgt hergestellt werden :

Beispiel 5

4-(2.3-Epoxy-propoxy)-3-(pyrazol-5-yl) methylen-indolinon (2)

4.1 g 4-(2.3-Epoxy-propoxy) indolinon (2) (Beispiel 6) werden in 40 ml Dimethylsulfoxid geloest, mit 2.0 g Pyrazol-5-aldehyd und 2.8 ml Triethylamin versetzt und 2 d bei Raumtemperatur geruehrt. Die Reaktionsmischung wird mit 150 ml Wasser versetzt und mit Essigester extrahiert. Nach Trocknen mit Natriumsulfat und Einengen kristallisieren 3.9 g der Titelverbindung vom Schmp. 229 °C aus, d. s. 68 % d. Th.

Analog erhaelt man :

(Siehe Tabelle Seite 18 f.)

| Bezeichnung | Ausbeute % |
|---|---|
| a) 4-(2.3-Epoxy-propoxy)-3-(2-hydroxybenzyliden)indolinon(2)<br>172-174 / Essigester<br>aus<br>4-(2.3-Epoxy-propoxy)indolinon(2)<br>(Beisp. 6)<br>und<br>Salicylaldehyd | 40 |

Das zur Herstellung in den Beispielen 3, 3a, 5 und 5a, benoetigte Epoxyd kann wie folgt hergestellt werden :

## Beispiel 6

4-(2.3-Epoxy-propoxy) indolinon (2)

Eine Loesung von 28.2 g 2-(2.3-Epoxy-propoxy)-6-nitrophenylessigsaeureethylester in 300 ml Methanol wird mit 3 ml Raney-Nickel versetzt und bei 1 bar Wasserstoffdruck hydriert. Nach Absaugen des Katalysators wird eingeengt und der Rueckstand in 300 ml Ether geloest, vom Unloeslichem abfiltiert und mit 12 ml Essigsaeure versetzt. Nach Ruehren ueber Nacht wird abgesaugt und mit Ether gewaschen. Es werden 13.1 g 4-(2.3-Epoxy-propoxy)-indolinon (2) vom Schmp. 164-166 °C erhalten, d. s. 64 % d. Th.

## Beispiel 7

4-(2-Hydroxy-3-isopropylamino-propoxy)-3-(2-hydroxybenzyl)-indolinon (2)-benzoat

5.8 g 4-(2-Hydroxy-3-isopropylamino-propoxy)-3-(2-hydroxy-benzyliden) indolinon (2) (Beispiel 1a) werden in 150 ml Methanol geloest, mit 15 ml Triethylamin versetzt und ueber 1 g 10 proz. Palladiumkohle bei Raumtemperatur und 1 bar Wasserstoff hydriert. Nach Absaugen des Katalysators wird im Vakuum abdestilliert, in Essigester geloest und mit der aequivalenten Menge Benzoesauere versetzt. Nach Absaugen verbleiben 2.5 g der Titelverbindung vom Schmp. 198-202 °C, d. s. 34 % d. Th.

Analog Beispiel 7 werden erhalten :

| Bezeichnung<br>Schmelzpunkt C° / Lösungsmittel | Ausbeute % |
|---|---|
| a) 4-(2-Hydroxy-3-isopropylamino-propoxy)-3-(3.4-dimethoxybenzyl)indolinon(2)-benzoat<br>82-84 / Essigester<br>aus<br>4-(2-Hydroxy-3-isopropylamino-propoxy)-3-(3.4-dimethoxybenzyliden)indolinon(2)<br>(Beisp. 1c) | 74 |
| b) 4-<2-Hydroxy-3-[2-(2-methoxyphenoxy)ethylamino]propoxy>-3-(3.4-dimethoxybenzyl)-indolinon(2)-oxalat<br>93-95 / Methanol<br>aus<br>4-<2-Hydroxy-3-[2-(2-methoxyphenoxy)-N-benzylethylamino]propoxy>-3-(3.4-dimethoxybenzyliden)indolinon(2) | 50 |

(Fortsetzung)

| Bezeichnung<br>Schmelzpunkt C° / Lösungsmittel | Ausbeute<br>% |
|---|---|
| c) 4-<2-Hydroxy-3-[2-(2-methoxyphenoxy)ethyl-amino]propoxy>-3-(2-hydroxybenzyl)indo-linon(2)-oxalat<br>156-158 / Methanol<br>aus<br>4-<2-Hydroxy-3-[2-(2-methoxyphenoxy)ethyl-amino]propoxy>-3-(2-hydroxybenzyliden)-indolinon(2) (Beispiel 1m) | 50 |
| d) 4-<2-Hydroxy-3-[2-(4-hydroxyphenoxy)ethyl-amino]propoxy>-3-(2-hydroxybenzyl)indo-linon(2)-cyclohexylsulfaminat<br>138-141 / Ethanol<br>aus<br>4-<2-Hydroxy-3-[2-(4-hydroxyphenoxy)ethyl-amino]propoxy>-3-(2-hydroxybenzyliden)indo-linon(2) (Beisp. 1p) | 30 |
| e) 4-<2-Hydroxy-3-[2-(4-hydroxyphenoxy)ethyl-amino]propoxy>-3-(pyrazol-5-yl-methyl)indo-linon(2)-fumarat<br>60 sint. / Isopropanol<br>aus<br>4-<2-Hydroxy-3-[2-(4-hydroxyphenoxy)ethyl-amino]propoxy>-3-(pyrazol-5-yl)methylen-indolinon(2) (Beisp. 1v) | 30 |
| f) 4-[2-Hydroxy-3-(2-phenoxyethylamino)-propoxy]-3-(pyrazol-5-yl-methyl)indo-linon(2)-fumarat<br>85 sint. / Isopropanol<br>aus<br>4-[2-Hydroxy-3-(2-phenoxyethylamino)-propoxy]-3-(pyrazol-5-yl)methylen-indolinon(2) (Beisp. 1u) | 63 |

Die zur Herstellung der Verbindung 7 b benoetigten Ausgangsstoffe koennen wie folgt erhalten werden :

3-(3.4-Dimethoxybenzyliden)-4-(2-hydroxy-3-[2-(2-methoxyphenoxy)-N-benzyl-ethylamino] propoxy) indolinon (2).

4.1 g 4-(2.3-Epoxy-propoxy) indolinon (2) (Beispiel 6) werden in 200 ml n-Butanol geloest und 5.2 g N-Benzyl-(2-methoxyphenoxy)-ethylamin zugegeben. Nach 3 d wird das Loesungsmittel im Vakuum entfernt. Es verbleiben 11.5 g Rohprodukt, das zur weiteren Umsetzung nicht gereinigt wurde.

11.5 g 4-(2-Hydroxy-3-[2-(2-methoxyphenoxy)-N-benzyl-ethylamino]-propoxy) indolinon (2) werden in 200 ml Ethanol geloest, 4.0 g 3.4-Dimethoxybenzaldehyd und 1 ml Piperidin zugegeben und anschließend 16 h zum Rueckfluß erhitzt. Nach Erkalten wird abgesaugt, mit Ethanol gewaschen und getrocknet. Es verbleiben 9.3 g der Titelverbindung vom Schmp. 151-155 °C, d. s. 76 % d. Th.

## Beispiel 8

4-(2-Hydroxy-3-isopropylamino-propoxy)-3-(2-hydroxy-4-methylsulfinyl-benzyliden) indolinon (2)-acetat

2.8 g 4-(2-Hydroxy-3-isopropylamino-propoxy)-3-(2-hydroxy-4-methylmercapto-benzyliden) indolinon (2) (Beisp. 1 d) werden in 50 ml Essigsaeure geloest und mit 0.85 ml 30 proz. Wasserstoffperoxid versetzt.

Nach 3 h Ruehren bei Raumtemperatur wird im Vakuum eingeengt und mit Wasser versetzt. Nach Abdekantieren wird der oelige Rueckstand in Ethanol geloest, mit 0.4 ml Eisessig versetzt und das kristalline Produkt abgesaugt. Es werden 2.0 g der Titelverbindung vom Schmp. 135-140 °C, d. s. 68 % d. Th. erhalten.

**Patentansprüche**

1. Indolinon-(2)-Derivate der allgemeinen Formel I

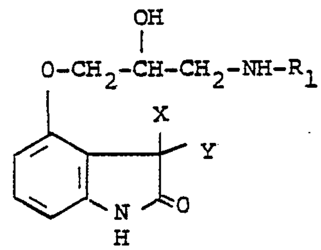

(I)

in welcher

$R_1$ eine $C_1$-$C_6$-Alkylgruppe oder eine Gruppe

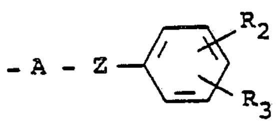

worin

A eine geradkettige oder verzweigte $C_2$-$C_4$-Alkylengruppe und

Z ein Sauerstoff- oder Schwefelatom,

$R_2$ und $R_3$,

die gleich oder verschieden sein koennen, jeweils Wasserstoff, Halogen, eine Hydroxygruppe, eine $C_2$-$C_6$-Alkanoylgruppe, eine $C_2$-$C_4$-Alkenylgruppe, eine $C_2$-$C_4$-Alkinylgruppe, eine $C_1$-$C_6$-Alkylgruppe, eine $C_1$-$C_6$-Alkoxygruppe, eine $C_2$-$C_4$-Alkenyloxygruppe, eine $C_2$-$C_4$-Alkinyloxygruppe, eine $C_1$-$C_6$-Alkylthiogruppe, eine $C_2$-$C_6$-Alkanoylamidogruppe oder eine Gruppe

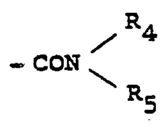

worin $R_4$ und $R_5$ gleich oder verschieden sind und Wasserstoff, einen $C_1$-$C_6$-Alkyl- oder $C_3$-$C_{10}$ Cycloalkylrest darstellen oder $R_4$ und $R_5$ gemeinsam einen gegebenenfalls durch ein Sauerstoff- oder Schwefelatom oder eine Gruppe $>N—R_6$, worin $R_6$ Wasserstoff oder eine $C_1$-$C_6$-Alkylgruppe bedeutet, unterbrochenen $C_2$-$C_8$-Alkylenrest darstellen, bedeuten,

X Wasserstoff und

Y Wasserstoff oder eine Gruppe

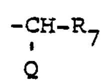

worin

Q Wasserstoff darstellt oder auch gemeinsam mit X eine Bindung bilden kann und $R_7$ einen Furan-, Thiophen-, Pyrrol-, Pyrazol-, Imidazol-, Triazol-, Tetrazol-, Imidazolin-, Pyridin-, Pyrimidin-, Uracil-, Indol- oder Indazol-Rest, der gegebenenfalls ein oder mehrfach durch Hydroxyl oder $C_1$-$C_6$-Alkyl substituiert sein kann oder einen Phenylrest, der gegebenenfalls ein oder mehrfach durch eine Hydroxygruppe, eine Mercaptogruppe, eine Carboxylgruppe, eine $C_1$-$C_6$-Alkoxycarbonylgruppe, eine $C_1$-$C_6$-Alkoxygruppe, eine $C_1$-$C_6$-Alkylthiogruppe, eine $C_1$-$C_6$-Alkylsulfinylgruppe, eine $C_1$-$C_6$-Alkylsulfonylgruppe, eine $C_2$-$C_6$-Alkanoylamidogruppe, eine $C_1$-$C_6$-Alkylsulfonylamidogruppe, eine Nitrogruppe, eine Aminogruppe, Halogen, eine $C_1$-$C_6$-Alkylgruppe, eine Methylendioxygruppe oder eine Cyanogruppe substituiert ist, darstellt, mit der Maßgabe, daß $R_1$ nicht eine $C_1$-$C_6$-Alkylgruppe sein kann, wenn X und Y gleichzeitig Wasserstoff bedeuten,

sowie deren pharmakologisch verträgliche Salze.

2. Verbindungen nach Anspruch 1, in denen $R_1$ eine $C_1$-$C_6$-Alkylgruppe, X und Q gemeinsam eine Bindung bilden und $R_7$ die angegebene Bedeutung hat.

3. Verbindungen nach Anspruch 1, in denen $R_1$ eine Gruppe

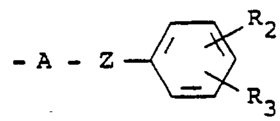

mit den angegebenen Bedeutungen für A, Z, $R_2$ und $R_3$ darstellt und X und Y die angegebenen Bedeutungen haben.

4. Verbindungen nach Anspruch 3, in denen A eine Ethylengruppe, Z Sauerstoff und $R_2$, $R_3$, X und Y die angegebenen Bedeutungen haben.

5. Verfahren zur Herstellung von Indolinon-(2)-Derivaten der allgemeinen Formel I

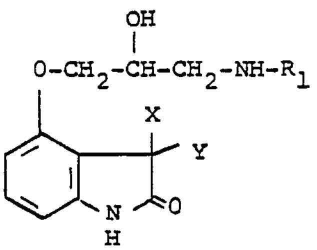

(I)

in welcher

$R_1$ eine $C_1$-$C_6$-Alkylgruppe oder eine Gruppe

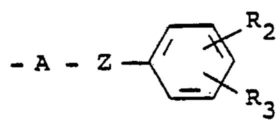

worin

A eine geradkettige oder verzweigte $C_2$-$C_4$-Alkylengruppe und

Z ein Sauerstoff- oder Schwefelatom,

$R_2$ und $R_3$, die gleich oder verschieden sein können, jeweils Wasserstoff, Halogen, eine Hydroxygruppe, eine $C_2$-$C_6$-Alkanoylgruppe, eine $C_2$-$C_4$-Alkenylgruppe, eine $C_2$-$C_4$-Alkinylgruppe, eine $C_1$-$C_6$-Alkylgruppe, eine $C_1$-$C_6$-Alkoxygruppe, eine $C_2$-$C_4$-Alkenyloxygruppe, eine $C_2$-$C_4$-Alkinyloxygruppe, eine $C_1$-$C_6$-Alkylthiogruppe, eine $C_2$-$C_6$-Alkanoylamidogruppe oder eine Gruppe

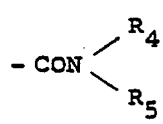

worin $R_4$ und $R_5$ gleich oder verschieden sind und Wasserstoff, einen $C_1$-$C_6$-Alkyl- oder $C_3$-$C_{10}$ Cycloalkylrest darstellen oder $R_4$ und $R_5$ gemeinsam einen gegebenenfalls durch ein Sauerstoff- oder Schwefelatom oder eine Gruppe $\geqslant N—R_6$, worin $R_6$ Wasserstoff oder eine $C_1$-$C_6$-Alkylgruppe bedeutet, unterbrochenen $C_2$-$C_8$-Alkylenrest darstellen, bedeuten,

X Wasserstoff und

Y Wasserstoff oder eine Gruppe

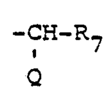

worin

Q Wasserstoff darstellt oder auch gemeinsam mit X eine Bindung bilden kann und $R_7$ einen Furan-, Thiophen-, Pyrrol-, Pyrazol-, Imidazol-, Triazol-, Tetrazol-, Imidazolin-, Pyridin-, Pyrimidin-, Uracil-, Indol- oder Indazol-Rest, der gegebenenfalls ein oder mehrfach durch Hydroxyl oder $C_1$-$C_6$-Alkyl substitutiert sein kann oder einen Phenylrest, der gegebenenfalls ein oder mehrfach durch eine Hydroxygruppe, eine Mercaptogruppe, eine Carboxylgruppe, eine $C_1$-$C_6$-Alkoxycarbonylgruppe, eine $C_1$-$C_6$-Alkoxygruppe, eine $C_1$-$C_6$-Alkylthiogruppe, eine $C_1$-$C_6$-Alkylsulfinylgruppe, eine $C_1$-$C_6$-Alkylsulfonylgruppe, eine $C_2$-$C_6$-Alkanoylamidogruppe, eine $C_1$-$C_6$-Alkylsulfonylamidogruppe, eine Nitrogruppe, eine Aminogruppe,

Halogen, eine $C_1$-$C_6$-Alkylgruppe, eine Methylendioxygruppe oder eine Cyanogruppe, substituiert ist, darstellt, mit der Maßgabe, daß $R_1$ nicht eine $C_1$-$C_6$ Alkylgruppe sein darf, wenn X und Y gleichzeitig Wasserstoff bedeuten,
sowie deren pharmakologisch verträglichen Salzen, dadurch gekennzeichnet, daß man in an sich bekannter Weise entweder

a) eine Verbindung der allgemeinen Formel II

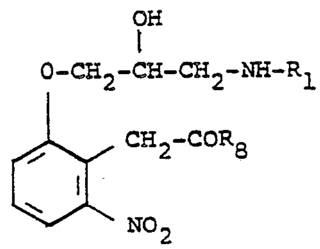

(II)

in welcher $R_1$ die angegebene Bedeutung hat und $R_8$, eine abspaltbare Gruppe darstellt, reduziert und cyclisiert und gewuenschtenfalls mit einer Verbindung der allgemeinen Formel III

$$O = CH - R_7 \quad \text{(III)}$$

in welcher $R_7$ die angegebene Bedeutung hat oder einem reaktiven Derivat hiervon, umsetzt oder

b) eine Verbindung der allgemeinen Formel IV

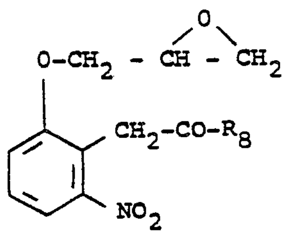

(IV)

in welcher $R_8$ die angegebene Bedeutung hat, reduziert und cyclisiert, und gewuenschtenfalls mit einer Verbindung der allgemeinen Formel III umsetzt, die dabei erhaltene Verbindung der allgemeinen Formel V

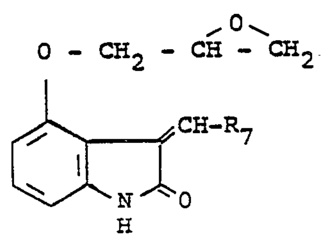

(V)

in welcher $R_7$ die angegebene Bedeutung hat mit einer Verbindung der allgemeinen Formel VI

$$H_2N - R_1 \quad \text{(VI)}$$

in welcher $R_1$ die angegebene Bedeutung hat umsetzt, gegebenenfalls anschließend eine Verbindung der allgemeinen Formel I, in der X und Q eine Bindung bilden, nach bekannten Methoden in eine Verbindung der allgemeinen Formel I, in der X und Q Wasserstoff bedeuten, umwandelt und gewuenschtenfalls in ein pharmakologisch vertraegliches Salz ueberfuehrt.

6. Arzneimittel, enthaltend eine Verbindung gemäß Anspruch 1, 2, 3 oder 4 sowie übliche Träger- und Hilfsstoffe.

7. Verbindungen gemäß Anspruch 1, 2, 3 oder 4 zur Herstellung von Arzneimitteln für die Behandlung von Herz- und Kreislauferkrankungen.

8. Zwischenprodukte der allgemeinen Formel V

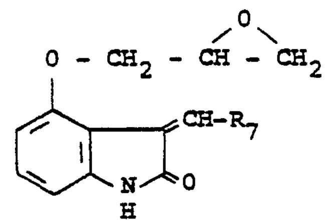

(V)

in der

$R_7$ einen Furan-, Thiophen-, Pyrrol-, Pyrazol-, Imidazol-, Triazol-, Tetrazol-, Imidazolin-, Pyridin-, Pyrimidin-, Uracil-, Indol- oder Indazol-Rest, der gegebenenfalls ein- oder mehrfach durch Hydroxyl oder $C_1$-$C_6$-Alkyl substitutiert sein kann oder einen Phenylrest, der gegebenenfalls ein- oder mehrfach durch eine Hydroxygruppe, eine Mercaptogruppe, eine Carboxylgruppe, eine $C_1$-$C_6$-Alkoxycarbonylgruppe, eine $C_1$-$C_6$-Alkoxygruppe, eine $C_1$-$C_6$-Alkylthiogruppe, eine $C_1$-$C_6$-Alkylsulfinylgruppe, eine $C_1$-$C_6$-Alkylsulfonylgruppe, eine $C_2$-$C_6$-Alkanoylamidogruppe, eine $C_1$-$C_6$-Alkylsulfonylamidogruppe, eine Nitrogruppe, eine Aminogruppe, Halogen, eine $C_1$-$C_6$-Alkylgruppe, eine Methylendioxygruppe oder eine Cyanogruppe substitutiert ist, darstellt.

9. Verbindungen gemäß Anspruch 8, in denen $R_7$ einen Imidazol-, Triazol-, Imidazolinon-, Pyrrol-, Pyrazol-, Pyridin-, Thiophen-, Indol-, Indazol-, Uracil- oder Phenyl-Rest bedeuten, der durch die angegebenen Gruppen substituiert sein kann.

10. Verfahren zur Herstellung von Verbindungen der allgemeinen Formel V

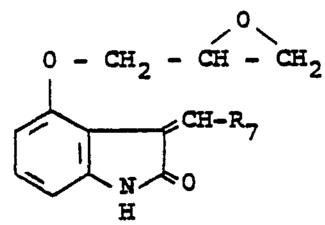

(V)

in der

$R_7$ einen Furan-, Thiophen-, Pyrrol-, Pyrazol-, Imidazol-, Triazol-, Tetrazol-, Imidazolin-, Pyridin-, Pyrimidin-, Uracil-, Indol- oder Indazol-Rest, der gegebenenfalls ein- oder mehrfach durch Hydroxyl oder $C_1$-$C_6$-Alkyl substituiert sein kann oder einen Phenylrest, der gegebenenfalls ein- oder mehrfach durch eine Hydroxygruppe, eine Mercaptogruppe, eine Carboxylgruppe, eine $C_1$-$C_6$-Alkoxycarbonylgruppe, eine $C_1$-$C_6$-Alkoxygruppe, eine $C_1$-$C_6$-Alkyl-thiogruppe, eine $C_1$-$C_6$-Alkylsulfinylgruppe, eine $C_1$-$C_6$-Alkysulfonylgruppe, eine $C_2$-$C_6$-Alkanoylmidogruppe, eine $C_1$-$C_6$-Alkylsulfonylamidogruppe, eine Nitrogruppe, eine Aminogruppe, Halogen, eine $C_1$-$C_6$-Alkylgruppe, eine Methylendioxygruppe oder eine Cyanogruppe substitutiert ist, darstellt, dadurch gekennzeichnet, daß man eine Verbindung der allgemeinen Formel VI

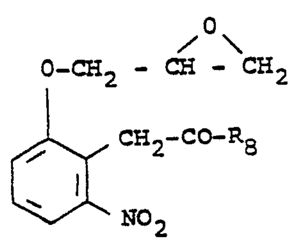

(IV)

in der $R_8$ eine abspaltbare Gruppe darstellt, reduziert und cyclisiert und mit einer Verbindung der allgemeinen Formel III

$$O = CH - R_7 \quad \text{(III)}$$

in welcher $R_7$ die angegebene Bedeutung hat oder einem reaktiven Derivat hervon umsetzt.

**Claims**

1. Indolin-2-one derivatives of the general formula I

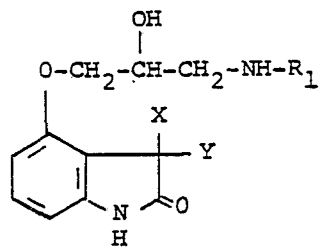

(I)

in which

$R_1$ signifies a $C_1$-$C_6$ alkyl group or a group

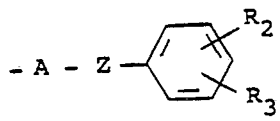

wherein

A signifies a straight-chained or branched $C_2$-$C_4$ alkylene group and

Z an oxygen or sulphur atom,

$R_2$ and $R_3$, which can be the same or different, each signify hydrogen, halogen, a hydroxyl group, a $C_2$-$C_6$ alkanoyl group, a $C_2$-$C_4$ alkenyl group, a $C_2$-$C_4$ alkynyl group, a $C_1$-$C_6$ alkyl group, a $C_1$-$C_6$ alkoxy group, a $C_2$-$C_4$ alkenyloxy group, a $C_2$-$C_4$ alkynyloxy group, a $C_1$-$C_6$ alkylthio group, a $C_2$-$C_6$ alkanoylamino group or a group

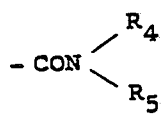

wherein $R_4$ and $R_5$ are the same or different and represent hydrogen, a $C_1$-$C_6$ alkyl or $C_3$-$C_{10}$ cycloalkyl radical or $R_4$ and $R_5$ together signify a $C_2$-$C_8$ alkylene radical optionally interrupted by an oxygen or sulphur atom or a group $\geq N—R_6$, wherein $R_6$ signifies hydrogen or a $C_1$-$C_6$ alkyl radical,

X hydrogen and

Y hydrogen or a group

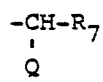

wherein

Q represents hydrogen or also, together with X, can form a bond and $R_7$ represents a furan, thiophene, pyrrole, pyrazole, imidazole, triazole, tetrazole, imidazoline, pyridine, pyrimidine, uracil, indole or indazole radical which can be optionally substituted one or more times by hydroxyl or $C_1$-$C_6$ alkyl or a phenyl radical which is optionally substituted one or more times by a hydroxyl group, a mercapto group, a carboxyl group, a $C_1$-$C_6$ alkoxycarbonyl group, a $C_1$-$C_6$ alkoxy group, a $C_1$-$C_6$ alkylthio group, a $C_1$-$C_6$ alkylsulphinyl group, a $C_1$-$C_6$ alkylsulphonyl group, a $C_2$-$C_6$ alkanoylamido group, a $C_1$-$C_6$ alkylsulphonylamido group, a nitro group, an amino group, halogen, a $C_1$-$C_6$ alkyl group, a methylenedioxy group or a cyano group, with the proviso that $R_1$ cannot be a $C_1$-$C_6$ alkyl radical when X and X simultaneously signify hydrogen,

as well as their pharmacologically acceptable salts.

2. Compounds according to claim 1, in which $R_1$ is a $C_1$-$C_6$ alkyl group, X and Q together form a bond and $R_7$ has the given meaning.

3. Compounds according to claim 1, in which $R_1$ represents a group

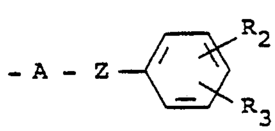

with the given meanings for A, Z, $R_2$ and $R_3$ and X and Y have the given meanings.

4. Compounds according to claim 3, in which A is an ethylene radical, Z oxygen and $R_2$, $R_3$, X and Y have the given meanings.

5. Process for the preparation of indolin-2-one derivatives of the general formula I

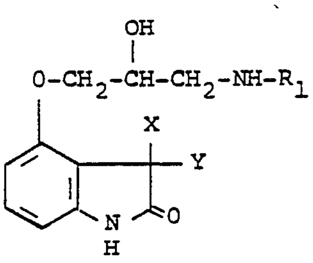

in which $R_1$ signifies a $C_1$-$C_6$ alkyl group or a group

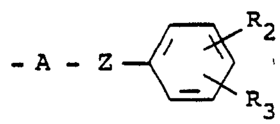

wherein

A signifies a straight-chained or branched $C_2$-$C_4$ alkylene group and

Z an oxygen or sulphur atom,

$R_2$ and $R_3$, which can be the same or different, each represent hydrogen, halogen, a hydroxyl group, a $C_2$-$C_6$ alkanoyl group, A $C_2$-$C_4$ alkenyl group, a $C_2$-$C_4$ alkynyl group, a $C_1$-$C_6$ alkyl group, a $C_1$-$C_6$ alkoxy group, a $C_2$-$C_4$ alkenyloxy group, a $C_2$-$C_4$ alkynyloxy group, a $C_1$-$C_6$ alkylthio group, a $C_2$-$C_6$ alkanoylamido group or a group

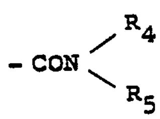

wherein $R_4$ and $R_5$ are the same or different and represent hydrogen, a $C_1$-$C_6$ alkyl or $C_3$-$C_{10}$ cycloalkyl radical or $R_4$ and $R_5$ together signify a $C_2$-$C_8$ alkylene radical optionally interrupted by an oxygen or sulphur atom or a group $>N—R_6$, wherein $R_6$ signifies hydrogen or a $C_1$-$C_6$ alkyl group,

X hydrogen and

Y hydrogen or a group

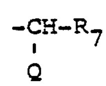

wherein

Q represents hydrogen or also, together with X, can form a bond and $R_7$ represents a furan, thiophene, pyrrole, pyrazole, imidazole, triazole, tetrazole, imidazoline, pyridine, pyrimidine, uracil, indole or indazole radical which can be optionally substituted one or more times by hydroxyl or $C_1$-$C_6$ alkyl or a phenyl radical which is optionally substituted one or more times by a hydroxyl group, a mercapto group, a carboxyl group, a $C_1$-$C_6$ alkoxycarbonyl group, a $C_1$-$C_6$ alkoxy group, a $C_1$-$C_6$ alkylthio group, $C_1$-$C_6$ alkylsulphinyl group, a $C_1$-$C_6$ alkylsulphonyl group, a $C_2$-$C_6$ alkanoylamido group, a $C_1$-$C_6$ alkylsulphonylamido group, a nitro group, an amino group, halogen, a $C_1$-$C_6$ alkyl group, a methylenedioxy group or a cyano group, with the proviso that $R_1$ cannot be a $C_1$-$C_6$ alkyl radical when X and Y simultaneously signify hydrogen,

as well as of their pharmacologically acceptable salts, characterized in that, in per se known manner, one either

a) reduces and cyclises a compound of the general formula II

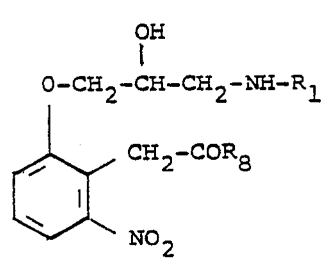

(II)

in which $R_1$ has the given meaning and $R_8$ represents a group which can be split off, and, if desired, reacts with a compound of the general formula III

$$O = CH - R_7 \qquad \text{(III)}$$

in which $R_7$ has the given meaning, or a reactive derivative thereof ; or

b) reduces and cyclises a compound of the general formula IV

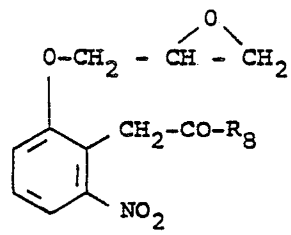

(IV)

in which $R_8$ has the given meaning, and, if desired, reacts with a compound of general formula III, reacts the compound thereby obtained of the general formula V

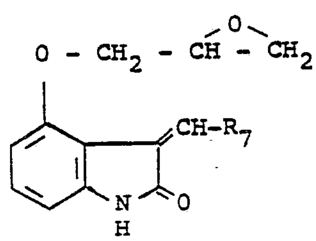

(V)

in which $R_7$ has the given meaning, with a compound of the general formula VI

$$H_2N - R_1 \qquad \text{(VI)}$$

in which $R_1$ has the given meaning, possibly subsequently converts a compound obtained of general formula I, in which X and Q form a bond, according to known methods into a compound of general formula I, in which X and Q signify hydrogen, and, if desired, converts into a pharmacologically acceptable salt.

6. Medicaments containing a compound according to claim 1, 2, 3 or 4, as well as usual carrier and adjuvant materials.

7. Compounds according to claim 1, 2, 3 or 4 for the production of medicaments for treating heart and circulatory diseases.

8. Intermediate products of the general formula V

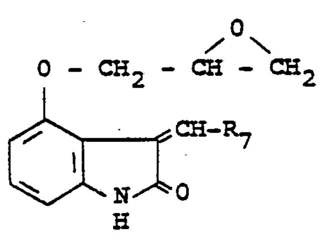

(V)

in which

$R_7$ represents a furan, thiophene, pyrrole, pyrazole, imidazole, triazole, tetrazole, imidazoline, pyridine, pyrimidine, uracil, indole or indazole radical which can be optionally substituted one or more times by hydroxyl or $C_1$-$C_6$ alkyl or a phenyl radical which is optionally substituted one or more times by a hydroxyl group, a mercapto group, a carboxyl group, a $C_1$-$C_6$ alkoxycarbonyl group, a $C_1$-$C_6$ alkoxy group, a $C_1$-$C_6$ alkylthio group, a $C_1$-$C_6$ alkylsulphinyl group, A $C_1$-$C_6$ alkylsulphonyl group, a $C_2$-$C_6$ alkanoylamido group, a $C_1$-$C_6$ alkylsulphonylamido group, a nitro group, an amino group, halogen, a $C_1$-$C_6$ alkyl group, a methylenedioxy group or a cyano group.

9. Compounds according to claim 8, in which $R_7$ signifies an imidazole, triazole, imidazoline, pyrrole, pyrazole, pyridine, thiophene, indole, indazole, uracil or phenyl radical, which can be substituted by the stated groups.

10. Process for the preparation of compounds of the general formula V

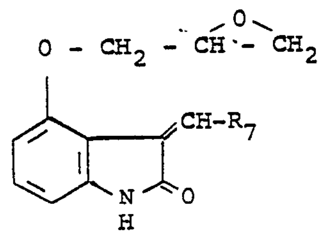

(V)

in which

$R_7$ represents a furan, thiophene, pyrrole, pyrazole, imidazole, triazole, tetrazole, imidazoline, pyridine, pyrimidine, uracil, indole or indazole radical which can be optionally substituted one or more times by hydroxyl or $C_1$-$C_6$ alkyl or a phenyl radical which is optionally substituted one or more times by a hydroxyl group, a mercapto group, a carboxyl group, a $C_1$-$C_6$ alkoxycarbonyl group, a $C_1$-$C_6$ alkoxy group, a $C_1$-$C_6$ alkylthio group, a $C_1$-$C_6$ alkylsulphinyl group, a $C_1$-$C_6$ alkylsulphonyl group, a $C_2$-$C_6$ alkanoylamido group, a $C_1$-$C_6$ alkylsulphonylamido group, a nitro group, an amino group, halogen, a $C_1$-$C_6$ alkyl group, a methylenedioxy group or a cyano group, characterised in that one reduces and cyclises a compound of the general formula IV

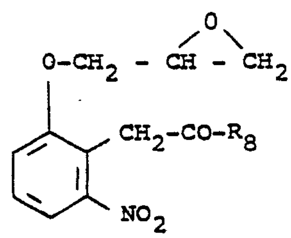

(IV)

in which $R_8$ represents a group which can be split off, and reacts with a compound of the general formula III

$$O = CH - R_7 \qquad (III)$$

in which $R_7$ has the given meaning, or with a reactive derivative thereof.

**Revendications**

1. Dérivés d'indolinone-(2) de la formule générale I,

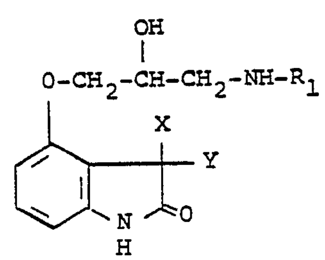

(I)

dans laquelle :

$R_1$ représente un groupe alkyle en $C_1$ à $C_6$ ou un groupe :

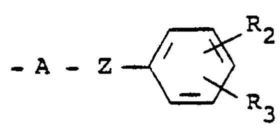

dans lequel :

A représente un groupe alkylène à chaîne droite ou ramifié en $C_2$ à $C_4$, et,

Z un atome d'oxygène ou de soufre,

$R_2$ et $R_3$, qui peuvent être semblables ou différents, représentent chacun l'hydrogène, un halogène, un groupe hydroxyle, un groupe alcanoyle en $C_2$ à $C_6$, un groupe alcényle en $C_2$ à $C_4$, un groupe alcynyle en $C_2$ à $C_4$, un groupe alkyle en $C_1$ à $C_6$, un groupe alcoxyle en $C_1$ à $C_6$, un groupe alcényloxyle en $C_2$ à

$C_4$, un groupe alcynyloxyle en $C_2$ à $C_4$, un groupe alkylthio en $C_1$ à $C_6$, un groupe alcanoylamide en $C_2$ à $C_6$ ou un groupe

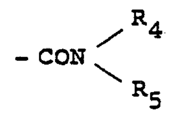

dans lequel $R_4$ et $R_5$ sont semblables ou différents et représentent l'hydrogène, un radical alkyle en $C_1$ à $C_6$ ou un radical cycloalkyle en $C_3$ à $C_{10}$, ou $R_4$ et $R_5$ représentent conjointement un radical alkylène en $C_2$ à $C_8$, éventuellement interrompu par un atome d'oxygène ou de soufre ou un groupe$\geq$N—$R_6$, dans lequel $R_6$ représente l'hydrogène ou un groupe alkyle en $C_1$ à $C_6$,

X l'hydrogène, et,

Y l'hydrogène ou un groupe

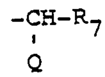

dans lequel :

Q représente l'hydrogène ou encore peut former une liaison conjointement avec X, et $R_7$ représente un radical furanne, thiophène, pyrrole, pyrazole, imidazole, triazole, tétrazole, imidazoline, pyridine, pyrimidine, uracile, indole ou indazole, qui peut éventuellement être substitué une ou plusieurs fois par des groupes hydroxyle ou alkyle en $C_1$ à $C_6$, ou un radical phényle qui peut éventuellement être substitué une ou plusieurs fois par un groupe hydroxyle, un groupe mercaptan, un groupe carboxyle, un groupe alcoxycarbonyle en $C_1$ à $C_6$, un groupe alcoxyle en $C_1$ à $C_6$, un groupe alkylthio en $C_1$ à $C_6$, un groupe alkylsulfinyle en $C_1$ à $C_6$, un groupe alkylsulfonyle en $C_1$ à $C_6$, un groupe alcanoylamide en $C_2$ à $C_6$, un groupe alkylsulfonylamide en $C_1$ à $C_6$, un groupe nitro, un groupe amine, un halogène, un groupe alkyle en $C_1$ à $C_6$, un groupe méthylènedioxy ou un groupe cyano, étant entendu que $R_1$ ne peut pas être un groupe alkyle en $C_1$ à $C_6$ quand X et Y représentent simultanément l'hydrogène, ainsi que leurs sels pharmacologiquement tolérés.

2. Composés selon la revendication 1, dans lesquels $R_1$ représente un groupe alkyle en $C_1$ à $C_6$, X et Q forment conjointement une liaison, et $R_7$ a la signification indiquée.

3. Composés selon la revendication 1, dans lesquels $R_1$ représente un groupe

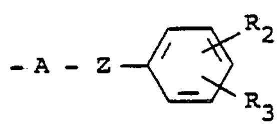

avec les significations indiquées pour A, Z, $R_2$ et $R_3$, et X et Y ont les significations indiquées.

4. Composés selon la revendication 3, dans lesquels A représente un groupe éthylène, Z l'oxygène, et $R_2$, $R_3$, X et Y ont les significations indiquées.

5. Procédé de préparation de dérivés d'indolinone-(2) de la formule générale I :

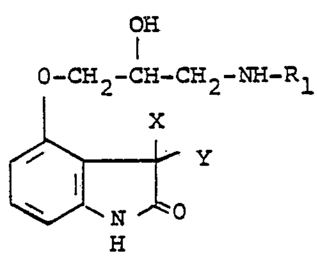

(I)

dans laquelle :

$R_1$ représente un groupe alkyle en $C_1$ à $C_6$ ou un groupe :

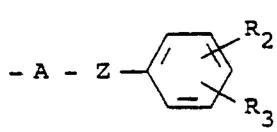

dans lequel :

A représente un groupe alkylène à chaîne droite ou ramifié en $C_2$ à $C_4$, et,

Z un atome d'oxygène ou de soufre,

$R_2$ et $R_3$, qui peuvent être semblables ou différents, représentent chacun l'hydrogène, un halogène, un groupe hydroxyle, un groupe alcanoyle en $C_2$ à $C_6$, un groupe alcényle en $C_2$-à $C_4$, un groupe alcynyle en $C_2$ à $C_4$, un groupe alkyle en $C_1$ à $C_6$, un groupe alcoxyle en $C_1$ à $C_6$, un groupe alcényloxyle en $C_2$ à $C_4$, un groupe alcynyloxyle en $C_2$ à $C_4$, un groupe alkylthio en $C_1$ à $C_6$, un groupe alcanoylamide en $C_2$ à $C_6$, ou un groupe

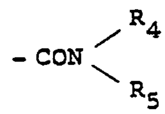

dans lequel $R_4$ et $R_5$ sont semblables ou différents et représentent l'hydrogène, un radical alkyle en $C_1$ à $C_6$ ou un radical cycloalkyle en $C_3$ à $C_{10}$, ou $R_4$ et $R_5$ représentent conjointement un radical alkylène en $C_2$ à $C_8$, éventuellement interrompu par un atome d'oxygène ou de soufre ou un groupe $>N—R_6$, dans lequel $R_6$ représente l'hydrogène ou un groupe alkyle en $C_1$ à $C_6$,

X l'hydrogène, et,

Y l'hydrogène ou un groupe

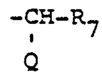

dans lequel :

Q représente l'hydrogène, ou encore peut former une liaison conjointement avec X, et $R_7$ représente un radical furanne, thiophène, pyrrole, pyrazole, imidazole, triazole, tétrazole, imidazoline, pyridine, pyrimidine, uracile, indole ou indazole, qui peut éventuellement être substitué une ou plusieurs fois par des groupes hydroxyle ou alkyle en $C_1$ à $C_6$, ou un radical phényle qui peut éventuellement être substitué une ou plusieurs fois par un groupe hydroxyle, un groupe mercaptan, un groupe carboxyle, un groupe alcoxycarbonyle en $C_1$ à $C_6$, un groupe alcoxyle en $C_1$ à $C_6$, un groupe alkylthio en $C_1$ à $C_6$, un groupe alkylsulfinyle en $C_1$ à $C_6$, un groupe alkylsulfonyle en $C_1$ à $C_6$, un groupe alcanoylamide en $C_2$ à $C_6$, un groupe alkylsulfonylamide en $C_1$ à $C_6$, un groupe nitro, un groupe amine, un halogène, un groupe alkyle en $C_1$ à $C_6$, un groupe méthylènedioxy ou un groupe cyano, étant entendu que $R_1$ ne peut pas être un groupe alkyle en $C_1$ à $C_6$ quand X et Y représentent simultanément l'hydrogène,

ainsi que de leurs sels pharmacologiquement tolérés, caractérisé par le fait que, de manière en elle-même connue, ou bien :

a) on réduit et on cyclise un composé de la formule générale II :

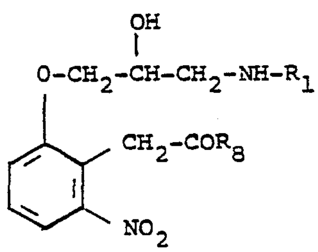

(II)

dans laquelle R a la signification indiquée, et $R_8$ représente un groupe séparable, et on le fait réagir, si on le désire, sur un composé de la formule générale III :

$$O = CH - R_7 \quad (III)$$

dans laquelle $R_7$ a la signification indiquée, ou un dérivé réactif de celui-ci ; ou bien,

b) on réduit et on cyclise un composé de la formule générale IV :

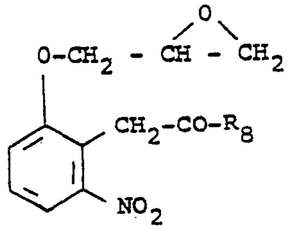

(IV)

dans laquelle $R_8$ a la signification indiquée,

et on le fait réagir, si on le désire, sur un composé de la formule générale III, on fait réagir le composé ainsi obtenu, de la formule générale V,

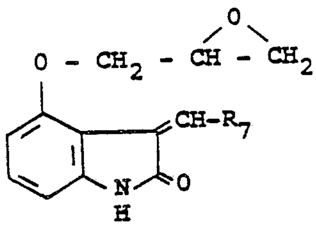

(V)

dans laquelle $R_7$ a la signification indiquée, sur un composé de la formule générale VI :

$$H_2N - R_1 \quad (VI)$$

dans laquelle $R_1$ a la signification indiquée, éventuellement, ensuite, on convertit un composé de la formule générale I, dans laquelle X et Q forment une liaison, selon des procédés connus, en un composé de la formule générale I dans laquelle X et Q représentent l'hydrogène, et, si on le désire, on le convertit en un sel pharmacologiquement toléré.

6. Médicament contenant un composé selon l'une des revendications 1, 2, 3 et 4, ainsi que des véhicules et adjuvants.

7. Composés selon l'une des revendications 1, 2, 3 et 4, pour la préparation de médicaments pour le traitement de maladies du cœur et de la circulation.

8. Produits intermédiaires de la formule générale V :

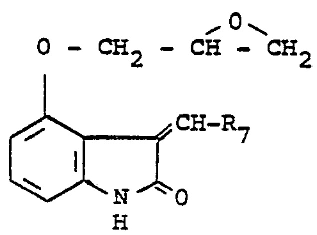

(V)

dans laquelle :

$R_7$ représente un radical furanne, thiophène, pyrrole, pyrazole, imidazole, triazole, tétrazole, imidazoline, pyridine, pyrimidine, uracile, indole ou indazole, qui peut être éventuellement substitué une ou plusieurs fois par des groupes hydroxyle ou alkyle en $C_1$ à $C_6$, ou un radical phényle qui est éventuellement substitué une ou plusieurs fois par un groupe hydroxyle, un groupe mercaptan, un groupe carboxyle, un groupe alcoxycarbonyle en $C_1$ à $C_6$, un groupe alcoxyle en $C_1$ à $C_6$, un groupe alkylthio en $C_1$ à $C_6$, un groupe alkylsulfinyle en $C_1$ à $C_6$, un groupe alkylsulfonyle en $C_1$ à $C_6$, un groupe alcanoylamide en $C_2$ à $C_6$, un groupe alkylsulfonylamide, un groupe nitro, un groupe amine, un halogène, un groupe alkyle en $C_1$ à $C_6$, un groupe méthylènedioxy ou un groupe cyano.

9. Composés selon la revendication 8, dans lesquels $R_7$ représente un radical imidazole, triazole, imidazolinone, pyrrole, pyrazole, pyridine, thiophène, indole, indazole, uracile ou phényle, qui peut être substitué par les groupes indiqués.

10. Procédé pour la préparation de composés de la formule générale V :

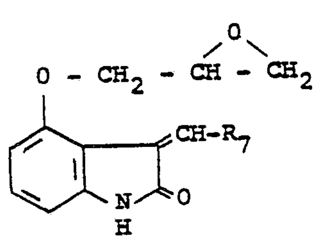

(V)

dans laquelle :

$R_7$ représente un radical furanne, thiophène, pyrrole, pyrazole, imidazole, triazole, tétrazole, imidazoline, pyridine, pyrimidine, uracile, indole ou indazole, qui peut être éventuellement substitué une ou plusieurs fois par des groupes hydroxyle ou alkyle en $C_1$ à $C_6$, ou un radical phényle qui est éventuellement substitué une ou plusieurs fois par un groupe hydroxyle, un groupe mercaptan, un groupe carboxyle, un groupe alcoxycarbonyle en $C_1$ à $C_6$, un groupe alcoxyle en $C_1$ à $C_6$, un groupe alkylthio en $C_1$ à $C_6$, un groupe alkylsulfinyle en $C_1$ à $C_6$, un groupe alkylsulfonyle en $C_1$ à $C_6$, un groupe

alcanoylamide en $C_2$ à $C_6$, un groupe alkylsulfonylamide, un groupe nitro, un groupe amine, un halogène, un groupe alkyle en $C_1$ à $C_6$, un groupe méthylènedioxy ou un groupe cyano, caractérisé par le fait que l'on réduit et que l'on cyclise un composé de la formule générale IV :

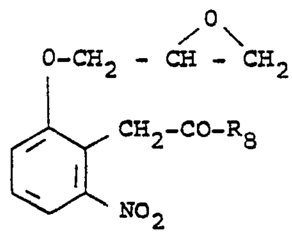

(IV)

dans laquelle $R_8$ représente un groupe séparable, et qu'on le fait réagir sur un composé de la formule générale III :

$$O = CH - R_7 \qquad \text{(III)}$$

dans laquelle $R_7$ a la signification indiquée, ou un dérivé réactif de celui-ci.